# EUROPÄISCHE PATENTSCHRIFT

(11) **EP 3 443 908 B1**
(45) Veröffentlichungstag und Bekanntmachung des Hinweises auf die Patenterteilung: **06.01.2021**
(21) Anmeldenummer: 17186236.0
(22) Anmeldetag: 15.08.2017
(51) Int. Cl.: A61B 6/04, A61B 6/10, A61B 6/00, B25J 9/16, G05B 19/4061

(54) **VERFAHREN ZUM BETREIBEN EINES RÖNTGENGERÄTS MIT EINEM GELENKARM UND RÖNTGENGERÄT MIT EINEM GELENKARM**
METHOD FOR OPERATING AN X-RAY DEVICE COMPRISING AN ARTICULATED ARM AND X-RAY DEVICE WITH AN ARTICULATED ARM
PROCÉDÉ DE FONCTIONNEMENT D'UN APPAREIL À RAYONS X POURVU D'UN BRAS ARTICULÉ ET APPAREIL À RAYON X POURVU D'UN BRAS ARTICULÉ

(43) Veröffentlichungstag der Anmeldung: 20.02.2019
(73) Patentinhaber: Siemens Healthcare GmbH, 91052 Erlangen (DE)
(72) Erfinder: Weingarten, Markus, 96049 Bamberg (DE)

(56) Entgegenhaltungen:
- WO-A1-2011/075232
- DE-A1-102008 046 348
- DE-A1-102008 057 142
- DE-A1-102016 201 701
- DE-A1-102016 209 576
- US-A- 5 485 502
- US-A1- 2009 097 612

## Beschreibung

Die US 548 5502 A offenbart, eine Computersteuerung eines mehrachsigen radiografischen Instruments. Diese wird verwendet, um einen Kontakt zwischen sich bewegenden Oberflächen des Instruments aufgrund von weichen Grenzwerten zu verhindern. Die weichen Grenzwerte werden aus einer Tabelle der gesetzlichen Zustände ermittelt, die alle Kombinationen der Achsbewegungen der Ausrüstung enthält und diese Kombinationen mit kontaktierenden oder berührungslosen Zuständen in Beziehung setzt. Die Legal- State-Tabelle kann verwendet werden, um effiziente Pfade zwischen einer Start- und einer Endposition abzubilden, und kann mit berührungslosen Wandlern erweitert werden, die eine Patientenbewegung erfassen können, die nicht in der Legal-State-Tabelle enthalten ist.

Die DE 10 2008 046 348 A1 offenbart ein Verfahren zur rechnergestützten Bahnplanung eines bewegbaren Geräts, insbesondere eines medizinischen Geräts, in einem Raumbereich. In dem Verfahren werden Bahnen des bewegbaren Geräts in geeigneter Weise mit Hilfe von Risikovolumina charakterisiert, welche bei der Bewegung des Geräts entlang einer jeweiligen Bahn das Risiko von Kollisionen mit anderen Objekten beschreiben. Aus den Risikovolumina wird ein Gesamtrisiko einer Kollision des bewegbaren Geräts mit Objekten im Raumbereich bestimmt und, basierend auf einem Optimierungsverfahren, welches als ein Optimierungsziel ein minimales Kollisionsrisiko enthält, werden rechnergestützt optimale Bahnen bestimmt. In einer bevorzugten Variante wird bei der Bahnplanung ferner die Anordnung von Sensoren zur Detektion von Objekten berücksichtigt, so dass das Verfahren ferner auch eine optimale Sensoranordnung ausgibt. Basierend auf den optimalen Bahnen bzw. der optimalen Sensoranordnung kann das Gerät im Betrieb geeignet bewegt werden, um hierdurch das Risiko von Kollisionen möglichst gering zu halten. Ein bevorzugter Anwendungsbereich ist die Bewegung von medizinischen Geräten, beispielsweise einer Röntgeneinrichtung in der Form eines C-Bogens.

Die DE 10 2008 057 142 A1 offenbart ein Verfahren zur rechnergestützten Bewegungsplanung eines Roboters, bei dem eine Trajektorie für die Bewegung eines dem Roboter zugeordneten Raumpunkts in einem ortsfesten Koordinatensystem geplant wird. Die Raumpositionen werden aus einer Vielzahl von Raumpositionen des Raumpunkts in jeweilige Konfigurationspositionen in einem Konfigurationsraum des Roboters basierend auf inverser Kinematik umgerechnet, wobei eine jeweilige Konfigurationsposition durch Achspositionen von einer oder mehreren rotatorischen und/oder translatorischen Bewegungsachsen des Roboters beschrieben wird. Die jeweiligen Konfigurationspositionen werden auf Kollisionen überprüft und es wird eine Trajektorie entlang von Raumpositionen des Raumpunkts gebildet, deren jeweilige Konfigurationspositionen kollisionsfrei sind. Durch eine Planung der Bewegung des Roboters in einem ortsfesten Koordinatensystem wird die Effizienz des Planungsverfahrens verbessert und die geplante Bewegung entspricht mehr den Erwartungen der Personen bzw. des Bedienpersonals im Umfeld des Roboters.

Die vorliegende Erfindung betrifft ein Verfahren zum Betreiben eines Röntgengeräts, insbesondere C-Bogen-Röntgengeräts, das einen Detektor oder eine Strahlungsquelle sowie einen Gelenkarm und einen Sockel aufweist, durch Vorgeben einer Startposition des Röntgengeräts bezüglich des Detektors oder der Strahlungsquelle, insbesondere des C-Bogens, und des Gelenkarms, Vorgeben einer Endposition des Röntgengeräts zumindest bezüglich des Detektors oder der Strahlungsquelle, insbesondere des C-Bogens, und Bewegen des Röntgengeräts von der Startposition in die Endposition. Darüber hinaus betrifft die vorliegende Erfindung ein Röntgengerät, insbesondere C-Bogen-Röntgengerät, mit einem Detektor oder einer Strahlungsquelle, einem Gelenkarm, an dessen distalem Ende der Detektor oder die Strahlungsquelle, insbesondere der C-Bogen, angeordnet ist, einem Sockel bzw. Stativ (gegebenenfalls auch zwei), an dem ein proximales Ende des Gelenkarms befestigt ist, und eine Bewegungseinrichtung, mittels der das Röntgengerät von einer bezüglich des Detektors oder der Strahlungsquelle, insbesondere des C-Bogens, und des Gelenkarms vorgegebenen Startposition in eine bezüglich des Detektors oder der Strahlungsquelle, insbesondere des C-Bogens, vorgegebene Endposition bewegbar ist.

Nachfolgend wird die Erfindung zur Vereinfachung ausschließlich anhand eines C-Bogen-Röntgengeräts beschrieben, ohne dass sie dadurch auf die Anwendung bei C-Bogen-Röntgengeräten beschränkt werden soll. Die Erfindung kann vielmehr auch bei Röntgengeräten angewendet werden, bei denen anstelle eines C-Bogens mit Detektor und Strahlungsquelle lediglich ein Detektor oder eine Strahlungsquelle an einem Gelenkarm angeordnet ist.

Ein C-Bogen-Röntgengerät besitzt üblicherweise einen Sockel, der auf dem Boden drehbar gelagert ist. An dem Sockel ist ein typischerweise in alle Raumrichtungen bewegbarer Gelenkarm mit seinem proximalen Ende angebracht. Am distalen Ende des Gelenkarms ist bewegbar ein C-Bogen befestigt. An dem einen Ende des C-Bogens befindet sich eine Röntgenstrahlungsquelle und an dem anderen Ende des C-Bogens befindet sich ein meist plattenförmiger Detektor.

C-Bogen-Röntgengeräte werden dazu benutzt, Objekte zu durchleuchten. Objekte können nicht nur Patienten, sondern auch Werkstücke wie etwa Turbinenflügel sein. Häufig sollen 3D-Bilder der durchleuchteten Objekte erstellt werden. Dazu ist es notwendig, dass der C-Bogen sehr viele unterschiedliche Stellungen gegenüber dem Objekt einnimmt. Gegebenenfalls sind auch dynamische Vorgänge, wie beispielsweise das Platzieren eines Katheters, mit dem C-Bogen-Röntgengerät zu überwachen. In diesem Fall muss der C-Bogen entsprechend nachgeführt werden. All diese Bewegungen des C-Bogen-Röntgengeräts werden dadurch erreicht, dass das C-Bogen-Röntgengerät mit mehreren Achsen versehen wird, die entsprechend angesteuert werden.

Ein solches C-Bogen-Röntgengerät weist häufig sechs (gegebenenfalls auch weniger oder mehr) Achsen auf. Diese Achsen sind während eines Bewegungsvorgangs komplex zu bewegen. Nicht selten ist ein derartiges C-Bogen-Röntgengerät dahingehend überbestimmt, dass eine bestimmte Position des C-Bogens ausgehend von einer Startposition durch mehrere verschiedene Bewegungstrajektorien beziehungsweise Pfade erreicht werden kann. Üblicherweise berechnet das System eigenständig einen Pfad, um von einer Startposition in eine vorgegebene Endposition zu gelangen.

C-Bogen-Röntgengeräte besitzen häufig Dimensionen von mehreren Metern in der Länge, in der Breite und in der Höhe. Entsprechend besitzen sie auch eine verhältnismäßig hohe Masse. Bei der automatisierten Bewegung des C-Bogen-Röntgengeräts kann daher eine deutliche Gefahr gegenüber Personen bestehen, die mit dem C-Bogen-Röntgengerät beziehungsweise in dessen Umgebung arbeiten. Vielfach ist zwar die Endposition eines C-Bogens den beteiligten Personen bekannt, aber sie kennen nicht den oder die Pfade, denen die einzelnen mit Gelenken verbundenen Komponenten des C-Bogen-Röntgengeräts bei der jeweiligen Bewegung folgen. Daher kommt es bei Bewegungen des C-Bogen-Röntgengeräts immer wieder zu Gefährdungen der beteiligten Personen.

Die Aufgabe der vorliegenden Erfindung besteht somit darin, die von einem C-Bogen-Röntgengerät ausgehende Gefahr bei dessen Bewegung für Personen und Gegenstände zu reduzieren.

Erfindungsgemäß wird diese Aufgabe durch ein Verfahren und ein C-Bogen-Röntgengerät gemäß den unabhängigen Ansprüchen gelöst. Vorteilhafte Weiterbildungen der Erfindung ergeben sich aus den Unteransprüchen.
Entsprechend der vorliegenden Erfindung wird demnach ein Verfahren zum Betreiben eines C-Bogen-Röntgengeräts, das einen C-Bogen mit Detektor und Strahlungsquelle sowie einen Gelenkarm und einen (ev. auch zwei) Sockel aufweist, bereitgestellt. Es soll also ein C-Bogen-Röntgengerät der eingangs erwähnten Art betrieben werden. Der Betrieb erfolgt typischerweise in einem Labor oder einem Operationssaal.

Zunächst erfolgt ein Vorgeben einer Startposition des C-Bogen-Röntgengeräts bezüglich des C-Bogens und des Gelenkarms. Dieses Vorgeben der Startposition kann durch entsprechende Sensorik automatisch erfolgen. Dabei zählen zu der Startposition beispielsweise die Stellung der einzelnen Glieder des C-Bogen-Röntgengeräts zueinander und gegebenenfalls auch die Stellung des C-Bogen-Röntgengeräts im umgebenden Raum. Vorliegend ist es von besonderem Interesse, diese Startposition hinsichtlich des C-Bogens und des Gelenkarms des C-Bogen-Röntgengeräts zu bestimmen beziehungsweise vorzugeben. Es ist also wichtig, wo sich der C-Bogen und der Gelenkarm zu Beginn einer Bewegung befinden, d.h. welche Startposition sie beziehungsweise das C-Bogen-Röntgengerät einnehmen.

Weiterhin erfolgt ein Vorgeben einer Endposition des C-Bogen-Röntgengeräts zumindest bezüglich des C-Bogens. Sinn und Zweck ist, dass sich der C-Bogen einschließlich Detektor und Strahlungsquelle in eine vorbestimmte Position z.B. relativ zu einem Objekt bewegt. Am Ende dieser Bewegung nimmt der C-Bogen die entsprechende Endposition ein. Diese Endposition kann automatisch oder manuell vorgegeben werden.

Außerdem erfolgt ein Bewegen des C-Bogen-Röntgengeräts von der Startposition in die Endposition. Da der Gelenkarm typischerweise in sich und der C-Bogen an dem Gelenkarm bewegbar sind, werden bei dem Bewegen des C-Bogen-Röntgengeräts entsprechend viele Achsen angesteuert beziehungsweise bewegt. In der Regel gibt es mehrere Wege beziehungsweise Pfade, auf denen der C-Bogen beziehungsweise das C-Bogen-Röntgengerät seine Endposition erreichen kann.

Entsprechend der vorliegenden Erfindung erfolgt nun ein automatisches Ermitteln mehrerer Pfade, welchen der Gelenkarm und der C-Bogen bei dem Bewegen von der Startposition in die Endposition folgen können. Es werden also insbesondere mehrere vollständige Pfade berechnet, auf denen sich der C-Bogen und der Gelenkarm bewegen können, um die Endposition zu erreichen. In einer Ausgestaltung handelt es sich bei einem Pfad um einen Weg, den beispielsweise der Schwerpunkt einer Komponente des C-Bogen-Röntgengeräts (C-Bogen oder Gelenkarm) ausgehend von der Startposition bis zur Endposition des C-Bogen-Röntgengeräts überstreicht. Gegebenenfalls stellt der Pfad aber auch ein komplexes geometrisches Gebilde dar, auf dem sich mehrere Komponenten des C-Bogen-Röntgengeräts bewegen. In einer besonders vorteilhaften Ausgestaltung stellt der Pfad den Raumbereich dar, innerhalb dessen sich der C-Bogen und der Gelenkarm bewegen. In einer einfacheren Ausgestaltung handelt es sich um eine 2D-Projektion dieses Raums.

Schließlich erfolgt ein Auswählen (manuell oder automatisch) eines der mehreren Pfade für das Bewegen des C-Bogen-Röntgengeräts. In einer einfachen Ausgestaltung wählt beispielsweise ein Nutzer des C-Bogen-Röntgengeräts einen der mehreren Pfade aus. Unter Umständen berücksichtigt der Auswählende andere Personen oder Objekte in der Umgebung des C-Bogen-Röntgengeräts bei seiner Auswahl. Auf diese Weise kann sichergestellt werden, dass von dem sich bewegenden C-Bogen-Röntgengerät weniger Gefahren für die Personen und Objekte in der Umgebung ausgehen. Insbesondere kann ein Pfad gewählt werden, bei dem jegliche Kollisionen vermieden werden.

Beispielsweise werden die mehreren Pfade grafisch zur Auswahl gestellt. Insbesondere kann also eine Benutzerschnittstelle mit einem Bildschirm vorgesehen sein, die die mehreren Pfade grafisch insbesondere in Bezug auf das C-Bogen-Röntgengerät beziehungsweise die erfassten Objekte darstellt. Gegebenenfalls werden die unterschiedlichen Pfade in zueinander verschiedenen Farben dargestellt. Gegebenenfalls kann der Nutzer einen jeweiligen Pfad mittels Berührung (falls es sich um einen Touchscreen handelt) oder mittels Tastatureingabe, Sprache, Gestik oder eine andere Bedienform auswählen.

Das Auswählen des Pfads für das Bewegen des C-Bogen-Röntgengeräts kann also manuell erfolgen. Damit lässt sich gewährleisten, dass nicht automatisch ein fester Pfad für die Bewegung verwendet wird. Vielmehr kann der Nutzer (zum Beispiel Operateur) selbst entscheiden, welchen Pfad das C-Bogen-Röntgengerät für seine Bewegung verwenden soll. Dadurch können zusätzliche Bedingungen wie beispielsweise die Position der Personen im Raum, die Geräte im Raum oder die Vorlieben des Operateurs, Berücksichtigung finden.

In einer Weiterbildung wird die Endposition des C-Bogen-Röntgengeräts auch bezüglich des Gelenkarms und/oder des Sockels vorgegeben. Die Endposition des C-Bogen-Röntgengeräts soll also nicht nur bezüglich des C-Bogens, sondern auch bezüglich einer oder mehrerer anderer Komponenten des C-Bogen-Röntgengeräts, hier des Gelenkarms beziehungsweise des Sockels, vorgegeben werden. Dadurch kann eine sehr spezifische Endposition des C-Bogen-Röntgengeräts erreicht werden.

Weiterhin kann vorgesehen sein, dass die Startposition des C-Bogen-Röntgengeräts auch bezüglich des Sockels vorgegeben wird. Dabei kann neben der relativen Position des Sockels zu dem Gelenkarm auch eine relative Position des Sockels zu einem Raum, in dem das C-Bogen-Röntgengerät aufgestellt ist (z.B. Operationssaal), berücksichtigt werden. Somit werden bei der Startposition sämtliche sich bewegende Hauptkomponenten des C-Bogen-Röntgengeräts berücksichtigt.

Vorzugsweise wird bei dem Ermitteln der mehreren Pfade ein Objekt in der Umgebung des C-Bogen-Röntgengeräts automatisch berücksichtigt. Befindet sich also beispielsweise ein von dem C-Bogen-Röntgengerät verschiedenes Objekt (z.B. Operationsschwester oder Anästhesiegerät) in der unmittelbaren Nähe des C-Bogen-Röntgengeräts, so kann dieses das Objekt mit geeigneter Sensorik erfassen. Wenn durch das Objekt der kollisionsfreie Bewegungsraum des C-Bogen-Röntgengeräts eingeschränkt ist, sollte dies beim Ermitteln der mehreren Pfade berücksichtigt werden. Das Gerät sollte dann nur diejenigen Pfade ermitteln beziehungsweise vorschlagen, die zu keiner Kollision führen. Für die Objekterfassung können Lichtschranken, Ultraschallsensoren, Kameras und dergleichen verwendet werden.

Des Weiteren kann vorgesehen sein, dass das Auswählen des Pfads für das Bewegen des C-Bogen-Röntgengeräts nach einem vom C-Bogen-Röntgengerät ausgeführten Lernvorgang automatisch erfolgt. Dies bedeutet, dass das C-Bogen-Röntgengerät frühere manuelle Auswahlvorgänge automatisch lernt. Wenn also beispielsweise mehrmals eine Herzklappenoperation durchgeführt wird, stehen der Chirurg und die Hilfspersonen in der Regel an immer den gleichen Orten. Das Gleiche gilt beispielsweise für Anästhesiegerätschaften. Das C-Bogen-Röntgengerät kann also einen von mehreren möglichen Pfaden als Standardpfad auswählen, sodass dieser beispielsweise auf einer Anzeige grafisch hervorgehoben wird. Der Nutzer des C-Bogen-Röntgengeräts kann dann durch eine einfache Bestätigung (z.B. per Taste) den automatisch ausgewählten Pfad bestätigen beziehungsweise die entsprechende Bewegung aktivieren.

Beispielsweise kann bei dem Auswählen einer der Pfade automatisch vorausgewählt und auf einer Bedienschnittstelle des C-Bogen-Röntgengeräts Korrekturmöglichkeiten zum Korrigieren des automatischen Pfads bereitgestellt werden. Es wird also beispielsweise von dem C-Bogen-Röntgengerät ein Standardpfad vorgeschlagen, und dieser Standardpfad kann nun korrigiert werden. Auf diese Weise hat der Nutzer vielfache Möglichkeiten, den Pfad zu beeinflussen. Gleichzeitig kann ihm ein aus Maschinensicht optimaler Pfad vorgeschlagen werden, welcher nur bezüglich Hindernissen abzuändern wäre.

Bei einer besonders bevorzugten Ausgestaltung wird das automatische Ermitteln mehrerer Pfade fortlaufend aktualisiert. Dies kann in mehrfacher Hinsicht von Vorteil sein. Zum einen kann das C-Bogen-Röntgengerät nach einer letzten Ermittlung der Pfade manuell verschoben worden sein. Dadurch ist es notwendig, die aktuelle Startposition neu zu ermitteln und ebenso die mehreren Pfade. Des Weiteren kann sich auch die Umgebung des C-Bogen-Röntgengeräts während des Betriebs verändert haben. So kann beispielsweise ein Beistelltisch verschoben worden sein, oder es hat sich eine Person in dem Raum bewegt. Dadurch kann das Berechnen vollkommen neuer Pfade erforderlich sein. Falls dieses automatische Ermitteln der Pfade kontinuierlich beziehungsweise fortlaufend erfolgt, stehen jederzeit mehrere aktuelle Pfade zur Verfügung.

Die oben genannte Aufgabe wird auch gelöst durch ein C-Bogen-Röntgengerät mit einem C-Bogen einschließlich Detektor und Strahlungsquelle, einem Gelenkarm, an dessen distalem Ende der C-Bogen angeordnet ist, einem Sockel, an dem ein proximales Ende des Gelenkarms befestigt ist, und einer Bewegungseinrichtung, mittels der das C-Bogen-Röntgengerät von einer bezüglich des C-Bogens und des Gelenkarms vorgegebenen Startposition in eine bezüglich des C-Bogens vorgegebene Endposition bewegbar ist sowie mit einer Recheneinrichtung zum automatischen Ermitteln mehrerer Pfade, welchen der Gelenkarm und der C-Bogen bei dem Bewegen von der Startposition in die Endposition folgen kann, und einer Auswahleinrichtung zum Auswählen eines der mehreren Pfade für das Bewegen des C-Bogen-Röntgengeräts.

Die oben im Zusammenhang mit dem erfindungsgemäßen Verfahren geschilderten Vorteile und Weiterbildungsmöglichkeiten gelten sinngemäß auch für das erfindungsgemäße C-Bogen-Röntgengerät. Dabei besitzen die entsprechenden Mittel des C-Bogen-Röntgengeräts die jeweiligen als Verfahrensmerkmale geschilderten Funktionen.

Die vorliegende Erfindung wird nun anhand der beigefügten Zeichnungen näher erläutert, in denen zeigen:
- FIG 1: eine schematische Ansicht eines C-Bogen-Röntgengeräts während eines Betriebs in einem Operationssaal, und
- FIG 2: ein Diagramm zum Ablauf eines erfindungsgemäßen Verfahrens.

Die nachfolgend näher geschilderten Ausführungsbeispiele stellen bevorzugte Ausführungsformen der vorliegenden Erfindung dar. Dabei ist zu beachten, dass die einzelnen Merkmale nicht nur in den geschilderten Merkmalskombinationen, sondern auch in Alleinstellung oder in anderen technisch sinnvollen Kombinationen realisiert werden können.

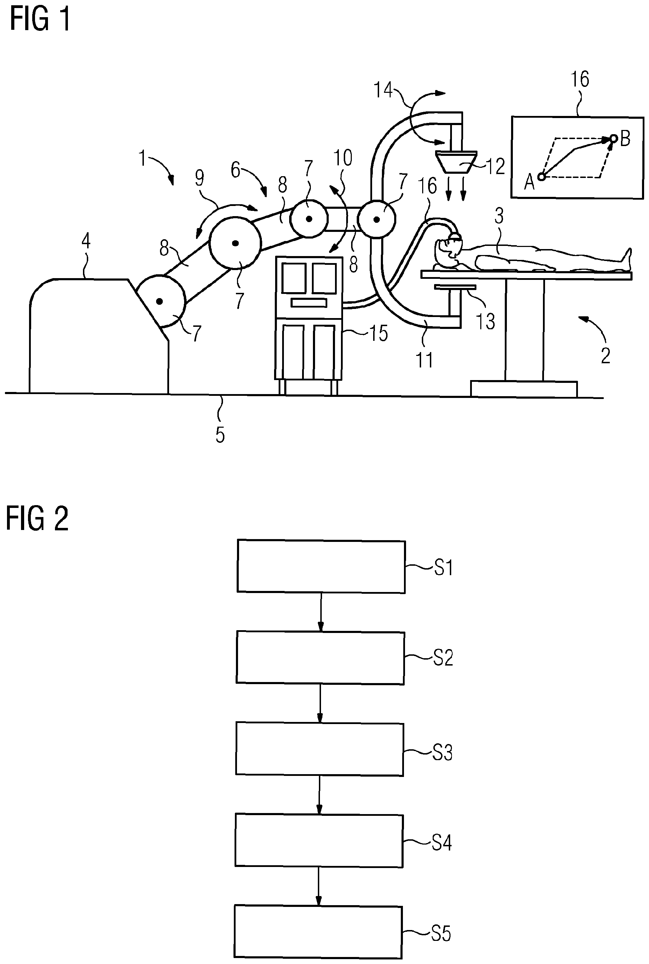

FIG 1 zeigt schematisch ein medizinisches System mit einem C-Bogen-Röntgengerät 1 und einem Patiententisch 2, auf dem sich ein Patient 3 befindet. Das C-Bogen-Röntgengerät 1 besitzt einen Sockel 4, der z.B. drehbar auf dem Boden 5 befestigt ist. Des Weiteren besitzt das C-Bogen-Röntgengerät 1 einen Gelenkarm 6, der mit seinem proximalen Ende an dem Sockel 4 schwenkbar und/oder drehbar gelagert ist. Der Gelenkarm 6 besitzt beispielsweise mehrere Gelenke 7 und zwischen jeweils zwei dieser Gelenken 7 einen jeweiligen Armabschnitt 8. Die Anzahl der Gelenke 7 beziehungsweise Armabschnitte 8 kann beliebig sein. Der Gelenkarm 6 erlaubt Bewegungen 9, 10 in verschiedene Raumrichtungen. Beispielsweise besitzt das gesamte C-Bogen-Röntgengerät 1 sechs Bewegungsachsen, um entsprechend komplexe Bewegungen durchzuführen.

Am distalen Ende des Gelenkarms 6 ist ein C-Bogen 11 angeordnet. An dem einen Ende des C-Bogens 11 befindet sich eine Röntgenstrahlungsquelle 12 und an dem anderen Ende ein Detektor 13. Der C-Bogen 11 kann beispielsweise eine Angulation 14 vollführen.

In dem Raum (z.B. Operationssaal) befindet sich außerdem in unmittelbarer Nähe des C-Bogen-Röntgengeräts 1 beziehungsweise des Patiententisches 2 mit dem Patienten 3 ein Anästhesieoder Beatmungsgerät 15. Ein Schlauch 17 führt von dem Beatmungsgerät 15 zu dem Patienten 3.

Das Beatmungsgerät 15 und der Schlauch 17 stehen stellvertretend für alle möglichen Objekte, die die Bewegung des C-Bogen-Röntgengeräts 1 einschränken können. Jedes Objekt in der Nähe des C-Bogen-Röntgengeräts 1 schränkt den Raum ein, in dem sich das Gerät kollisionsfrei bewegen kann. Als derartige Objekte gelten auch andere Geräte beziehungsweise Versorgungssysteme (einschließlich Kabel und Schläuche) sowie Personen.

Weiterhin ist an dem C-Bogen-Röntgengerät 1 oder in dem Raum (z.B. Operationssaal) eine Bedienschnittstelle 16 angeordnet. Diese Bedienschnittstelle 16 informiert beispielsweise über die Bewegungsmöglichkeiten des C-Bogen-Röntgengeräts 1.

Im vorliegenden Fall soll sich das C-Bogen-Röntgengerät 1 von einem ersten Punkt A zu einem zweiten Punkt B bewegen. Dabei wird im einfachsten Fall beispielsweise nur der Mittelpunkt zwischen Röntgenstrahlungsquelle 12 und Detektor 13 als maßgeblicher Punkt betrachtet. Dieser Punkt soll also von Punkt A bis Punkt B wandern. Das System schlägt nun beispielsweise drei Pfade vor, wie der Punkt B ausgehend von dem Punkt A erreicht werden kann, wie dies in der Bedienschnittstelle 16 (z.B. Touch-Monitor) von FIG 1 dargestellt ist. Gegebenenfalls werden auch nur zwei Pfade oder auch mehr als drei Pfade vorgeschlagen. Der Operateur entscheidet sich für einen dieser mehreren Pfade durch eine entsprechende Eingabe in die Bedienschnittstelle 16. Beispielsweise tippt er einen der eingezeichneten Pfade an. Dadurch wählt er vorzugsweise nicht nur den gewünschten Pfad aus, sondern aktiviert auch das C-Bogen-Röntgengerät 1 zur entsprechenden Bewegung.

Durch die Auswahl des Pfads berücksichtigt der Nutzer beziehungsweise Operateur die Objekte beziehungsweise Personen in der Umgebung des C-Bogen-Röntgengeräts 1. So kann eine kollisionsfreie Bewegung von einer Startposition A des C-Bogen-Röntgengeräts 1 in eine Endposition B erfolgen.

Anhand von FIG 2 lässt sich der Verfahrensablauf zum Betreiben eines C-Bogen-Röntgengeräts detaillierter erläutern. Zunächst erfolgt in einem Schritt S1 ein Vorgeben einer Startposition des C-Bogen-Röntgengeräts 1. Insbesondere wird bei der Startposition nicht nur der C-Bogen 11 selbst, sondern auch der Gelenkarm 6 berücksichtigt. Aufgrund der Vielzahl an Achsen vollführt der Gelenkarm 6 komplexe Bewegungen und muss aufgrund seiner großen körperlichen Ausdehnungen bei der Bewegung des C-Bogen-Röntgengeräts 1 ebenso beachtet werden wie der C-Bogen 11 selbst. Das Vorgeben der Startposition erfolgt in der Regel automatisch durch die Sensorik des C-Bogen-Röntgengeräts 1, da die Gelenkstellungen der Gelenke 7 automatisch erfasst werden. Mit den Abmessungen der Armabschnitte 8, der Gelenke 7, aber auch des Sockels 4 und des C-Bogens 11 kann dann die jeweilige Startposition des C-Bogen-Röntgengeräts 1 exakt ermittelt beziehungsweise vorgegeben werden.

In einem anschließenden Schritt S5 wird die Endposition des C-Bogen-Röntgengeräts 1 zumindest bezüglich des C-Bogens 11 vorgegeben. Der C-Bogen 11 soll ja in eine bestimmte Position gebracht werden, um dort für eine gewünschte Röntgenaufnahme bereit zu stehen. Wie in dieser Stellung des C-Bogens 11 der Gelenkarm 6 geformt ist, ist für die eigentliche Röntgenaufnahme sekundär. Für die gefahrlose Bewegung des C-Bogen-Röntgengeräts 1 von einer Startposition A in eine Endposition B ist jedoch auch die Bewegung und Form des Gelenkarms 6 ausschlaggebend.

Um nun festzustellen, wie das C-Bogen-Röntgengerät 1 zumindest bezüglich des C-Bogens 11 in die gewünschte Endposition bewegt werden kann, erfolgt gemäß Schritt S3 ein automatisches Ermitteln mehrerer Pfade, welchen der Gelenkarm 6 und der C-Bogen 11 bei dem Bewegen von der Startposition A in die Endposition B folgen kann. Vorzugsweise werden diese mehreren Pfade auf einem Monitor oder einer anderen Bedienschnittstelle 16 grafisch dargestellt. In einem anschließenden Schritt S4 wird einer der mehreren ermittelten Pfade für das Bewegen des C-Bogen-Röntgengeräts 1 ausgewählt. Dazu wird beispielsweise derjenige Pfad auf der Bedienschnittstelle 16 angetippt, der gewünscht ist. Insbesondere wird ein solcher Pfad gewählt, der garantiert, dass das C-Bogen-Röntgengerät 1 bei seiner Bewegung nicht mit einem Objekt oder einer Person kollidiert.

Schließlich erfolgt gemäß Schritt S5 ein Bewegen des C-Bogen-Röntgengeräts 1 entlang des gewählten Pfads. Dieses Bewegen wird beispielsweise durch ein manuelles Bestätigen oder Auswählen eines der mehreren Pfade aktiviert.

In einer Weiterbildung wird auf der Benutzerschnittstelle 16 nicht nur ein Pfad als Linie (z.B. des Mittelpunkts zwischen Röntgenquelle 12 und Detektor 13), sondern als Fläche oder Raum dargestellt, den das C-Bogen-Röntgengerät 1 bei seiner Bewegung überstreicht beziehungsweise durchläuft. Dadurch wird jeder Pfad zu einem zweidimensionalen oder dreidimensionalen Gebilde. Ein Nutzer kann somit besser überblicken, welche Bereiche beziehungsweise Räume das C-Bogen-Röntgengerät 1 für seine Bewegungen braucht. Gegebenenfalls werden dabei zusätzlich die Personen und/oder Objekte im Bereich des C-Bogen-Röntgengeräts 1 erfasst und ebenfalls auf der Benutzerschnittstelle 16 grafisch dargestellt. Vorzugsweise werden aber nur solche Pfade für die Bewegung vorgeschlagen, die von vornherein eine Kollision mit Objekten beziehungsweise Personen vermeiden.

Gegebenenfalls lernt das System auch, bestimmte Pfade als Standardpfade vorzuschlagen. So lassen sich beispielsweise die Pfade auch personalisieren. Befindet sich bei einem bestimmten Operateur die Anästhesiegerätschaft beispielsweise immer links vom Patiententisch, so kann dies bei dem Ermitteln der mehreren Pfade beziehungsweise bei Vorgeben eines Standardpfads Berücksichtigung finden.

Das geschilderte Verfahren ist vorzugsweise dynamisch ausgebildet. Zieht beispielsweise der Arzt den Patiententisch 2 zurück, so wird eine andere Trajektorie beziehungsweise ein anderer Pfad für die Bewegung des C-Bogen-Röntgengeräts 1 ermöglicht, was an der Benutzerschnittstelle 16 auch instantan angezeigt werden sollte. Daher erfolgt das automatische Ermitteln mehrerer Pfade vorzugsweise fortlaufend.

Das erfindungsgemäße Verfahren zum Betreiben eines C-Bogen-Röntgengeräts lässt sich nicht nur dazu verwenden, das C-Bogen-Röntgengerät von einer Untersuchungsposition in eine andere Untersuchungsposition zu bewegen. Vielmehr kann das Verfahren auch dazu herangezogen werden, um beispielsweise das C-Bogen-Röntgengerät von einer Parkposition, bei der sich der C-Bogen 11 beispielsweise an der Decke befindet, in eine Untersuchungsposition zu bewegen. Auch dabei ist es wichtig, die aktuelle Situation zu berücksichtigen, um Kollisionen zu vermeiden.

## Patentansprüche

1. Verfahren zum Betreiben eines Röntgengeräts (1), das einen Detektor (13) oder eine Strahlungsquelle (12) oder einen C-Bogen (11) mit Detektor (13) und Strahlungsquelle (12) sowie einen Gelenkarm (6) und einen Sockel ,(4) aufweist, durch
- Vorgeben einer Startposition (S1) des Röntgengeräts (1) bezüglich des C-Bogens (11) oder des Detektors (13) oder der Strahlungsquelle (12) und des Gelenkarms (6),
- Vorgeben einer Endposition (S2) des Röntgengeräts (1) zumindest bezüglich des C-Bogens (11) oder des Detektors (13) oder der Strahlungsquelle (12) und
- Bewegen (S5) des Röntgengeräts (1) von der Startposition in die Endposition,
**gekennzeichnet durch**
- automatisches Ermitteln mehrerer Pfade (S3), welchen der Gelenkarm (6) und der C-Bogen (11) oder der Detektor (13) oder die Strahlungsquelle (12) bei dem Bewegen (S5) von der Startposition in die Endposition folgen kann,
- Graphisches Darstellen der mehreren Pfade zur Auswahl, und
- Manuelles Auswählen des Pfades (S4) für das Bewegen des Röntgengeräts (1).

2. Verfahren nach Anspruch 1, wobei die Endposition des Röntgengeräts (1) auch bezüglich des Gelenkarms (6) und/oder des Sockels (4) vorgegeben wird (S2).

3. Verfahren nach Anspruch 1 oder 2, wobei die Startposition des Röntgengeräts (1) auch bezüglich des Sockels (4) vorgegeben wird (S1).

4. Verfahren nach einem der vorhergehenden Ansprüche, wobei bei dem Ermitteln der mehreren Pfade (S3) ein Objekt (15, 16) in der Umgebung des Röntgengeräts (1) automatisch berücksichtigt wird.

5. Verfahren nach einem der vorhergehenden Ansprüche, wobei bei dem Auswählen (S4) einer der Pfade automatisch vorausgewählt wird und auf einer Bedienschnittstelle (16) des Röntgengeräts (1) Korrekturmöglichkeiten zum Korrigieren des automatischen Pfads bereitgestellt werden.

6. Verfahren nach einem der vorhergehenden Ansprüche, wobei das automatische Ermitteln mehrerer Pfade (S3) fortlaufend aktualisiert wird.

7. Röntgengerät (1) mit
- einem Detektor (13) oder einer Strahlungsquelle (12) oder einem C-Bogen (11) einschließlich Detektor (13) und Strahlungsquelle (12),
- einem Gelenkarm (6), an dessen distalem Ende der C-Bogen (11) oder der Detektor (13) oder die Strahlungsquelle (12) angeordnet ist,
- einem Sockel (4), an dem ein proximales Ende des Gelenkarms (6) befestigt ist, und
- einer Bewegungseinrichtung, mittels der das Röntgengerät von einer bezüglich des C-Bogens (11) oder des Detektors (13) oder der Strahlungsquelle (12) und des Gelenkarms (6) vorgegebenen Startposition in eine bezüglich des C-Bogens (11) oder des Detektors (13) oder der Strahlungsquelle (12) vorgegebene Endposition bewegbar ist,
**gekennzeichnet durch**
- eine Recheneinrichtung zum automatischen Ermitteln mehrerer Pfade, welchen der Gelenkarm (6) und der C-Bogen (11) oder der Detektor (13) oder die Strahlungsquelle (12) bei dem Bewegen von der Startposition in die Endposition folgen kann, und
- eine Benutzerschnittstelle (16)zum graphischen Darstellen der mehreren Pfade und zum manuellen Auswählen des Pfades für das Bewegen des Röntgengeräts (1).

## Claims

1. Method for operating an X-ray device (1) comprising a detector (13) or a radiation source (12) or a C-arm (11) with a detector (13) and a radiation source (12) and an articulated arm (6) and a base (4) by means of
- specification of a starting position (S1) of the X-ray device (1) with respect to the C-arm (11) or the detector (13) or the radiation source (12) and the articulated arm (6),
- specification of an end position (S2) of the X-ray device (1) at least with respect to the C-arm (11) or the detector (13) or the radiation source (12) and
- movement (S5) of the X-ray device (1) from the starting position into the end position,
**characterised by**
- automatic ascertainment of a plurality of paths (S3) that can be followed by the articulated arm (6) and the C-arm (11) or the detector (13) or the radiation source (12) on movement (S5) from the starting position into the end position,
- graphical depiction of the plurality of paths for selection, and
- manual selection of the path (S4) for the movement of the X-ray device (1)).

2. Method according to claim 1, wherein the end position of the X-ray device (1) is also specified (S2) with respect to the articulated arm (6) and/or the base (4).

3. Method according to claim 1 or 2, wherein the starting position of the X-ray device (1) is also specified (S1) with respect to the base (4).

4. Method according to one of the preceding claims, wherein the ascertainment of the plurality of paths (S3) automatically takes account of an object (15, 16) in the environment of the X-ray device (1).

5. Method according to one of the preceding claims, wherein during the selection (S4), one of the paths is automatically preselected and correction options for the correction of the automatic path are provided on an operator interface (16) of the X-ray device (1).

6. Method according to one of the preceding claims, wherein the automatic ascertainment of a plurality of paths (S3) is updated continuously.

7. X-ray device (1) with
- a detector (13) or a radiation source (12) or a C-arm (11) including a detector (13) and a radiation source (12),
- an articulated arm (6) on the distal end of which the C-arm (11) or the detector (13) or the radiation source (12) is arranged,
- a base (4) to which a proximal end of the articulated arm (6) is attached and
- a movement means by means of which the X-ray device can be moved from a starting position specified with respect to the C-arm (11) or the detector (13) or the radiation source (12) and the articulated arm (6) into an end position specified with respect to the C-arm (11) or the detector (13) or the radiation source (12),
**characterised by**
- a computing means for the automatic ascertainment of a plurality of paths that can be followed by the articulated arm (6) and the C-arm (11) or the detector (13) or the radiation source (12) on movement from the starting position into the end position and
- a user interface (16) for the graphical depiction of the plurality of paths and for the manual selection of the path for the movement of the X-ray device (1).

## Revendications

1. Procédé pour faire fonctionner un appareil ( 1 ) à rayons X, qui a un détecteur ( 13 ) ou une source ( 12 ) de rayonnement ou un arceau ( 11 ) en C, ayant un détecteur ( 13 ) et une source ( 12 ) de rayonnement, ainsi qu'un bras ( 6 ) articulé et un socle ( 4 ), par
- prescription d'une position ( S1 ) initiale de l'appareil ( 1 ) à rayons X, par rapport à l'arceau ( 11 ) en C ou au détecteur ( 13 ) ou à la source ( 12 ) de rayonnement et au bras ( 6 ) articulé,
- prescription d'une position ( S2 ) finale de l'appareil ( 1 ) à rayons X, au moins par rapport à l'arceau ( 11 ) en C ou au détecteur ( 13 ) ou à la source ( 12 ) de rayonnement et
- passage ( S5 ) de l'appareil ( 1 ) à rayons X de la position initiale à la position finale,
**caractérisé par**
- détermination automatique de plusieurs trajets ( S3 ), que le bras ( 6 ) articulé et l'arceau ( 11 ) en C ou le détecteur ( 13 ) ou la source ( 12 ) de rayonnement peut suivre lors du passage ( S5 ) de la position initiale à la position finale,
- représentation graphique des plusieurs trajets pour la sélection, et
- sélection manuelle du trajet ( S4 ) pour le déplacement de l'appareil ( 1 ) à rayons X.

2. Procédé suivant la revendication 1, dans lequel on prescrit ( S2 ) la position finale de l'appareil ( 1 ) à rayons X, également par rapport au bras ( 6 ) articulé et/ou au socle ( 4 ).

3. Procédé suivant la revendication 1 ou 2, dans lequel on prescrit ( S1 ) la position initiale de l'appareil ( 1 ) à rayons X, également par rapport au socle ( 4 ).

4. Procédé suivant l'une des revendications précédentes, dans lequel, lors de la détermination des plusieurs trajets ( S3 ), on prend en compte automatiquement un objet ( 15, 16 ) aux alentours de l'appareil ( 1 ) à rayons X.

5. Procédé suivant l'une des revendications précédentes, dans lequel, lors de la sélection ( S4 ), on sélectionne automatiquement l'un des trajets et l'on met à disposition, sur une interface ( 16 ) de l'appareil ( 1 ) à rayons X, des possibilités de correction pour corriger le trajet automatique.

6. Procédé suivant l'une des revendications précédentes, dans lequel on met à jour en continu la détermination automatique de plusieurs trajets ( S3 ).

7. Appareil ( 1 ) à rayons X, comprenant
- un détecteur ( 13 ) ou une source ( 12 ) de rayonnement ou un arceau ( 11 ) en C, incluant un détecteur ( 13 ) et une source ( 12 ) de rayonnement,
- un bras ( 6 ) articulé, à l'extrémité distale duquel est monté l'arceau ( 11 ) en C ou le détecteur ( 13 ) ou la source ( 12 ) de rayonnement,
- un socle ( 4 ), auquel une extrémité proximale du bras ( 6 ) articulé est fixée, et
- un dispositif de déplacement, au moyen duquel l'appareil à rayons X peut passer d'une position initiale prescrite, par rapport à l'arceau ( 11 ) en C ou au détecteur ( 13 ) ou à la source ( 12 ) de rayonnement et au bras ( 6 ) articulé, en une position finale prescrite, par rapport à l'arceau ( 11 ) en C ou au détecteur ( 13 ) ou à la source ( 12 ) de rayonnement,
**caractérisé par**
- un dispositif informatique de détermination automatique de plusieurs trajets, que le bras ( 6 ) articulé et l'arceau ( 11 ) en C ou le détecteur ( 13 ) ou la source ( 12 ) de rayonnement peut suivre lors du passage de la position initiale à la position finale, et
- une interface ( 16 ) d'utilisateur de représentation graphique des plusieurs trajets et de sélection manuelle du trajet pour le déplacement de l'appareil ( 1 ) à rayons X.
